# FASCICULE DE BREVET EUROPEEN

(11) **EP 3 474 954 B1**
(45) Date de publication et mention de la délivrance du brevet: **29.11.2023**
(21) Numéro de dépôt: 17731858.1
(22) Date de dépôt: 15.06.2017
(51) Int. Cl.: A61K 8/97, A61Q 19/08, A61K 36/36

(54) **PROCÉDÉ D'OBTENTION D'UN EXTRAIT D'ARMERIA MARITIMA ENRICHI EN DIGLYCOSIDES ET SON UTILISATION EN COSMÉTIQUE**
VERFAHREN ZUR HERSTELLUNG EINES MIT DIGLYCOSID ANGEREICHERTEN ARMERIA-MARITIMA-EXTRAKTS UND DESSEN VERWENDUNG IN DER KOSMETIK
METHOD FOR OBTAINING A DIGLYCOSIDE-ENRICHED ARMERIA MARITIMA EXTRACT, AND USE THEREOF IN COSMETICS

(30) Priorité: 23.06.2016 FR 1655868
(43) Date de publication de la demande: 01.05.2019
(73) Titulaire: Société d'Exploitation de Produits pour les Industries Chimiques SEPPIC, 75321 Paris Cedex 07 (FR); Centre National de la Recherche Scientifique, 75794 Paris Cedex 16 (FR); L'Universite de Strasbourg, 67000 Strasbourg (FR)
(72) Inventeur: GOURGUILLON, Lorène, 22260 Quemper Guezennec (FR); CATTUZZATO, Laetitia, 81100 Castres (FR)
(74) Mandataire: Air Liquide
(86) Numéro de dépôt international: PCT/EP2017/064685
(87) Numéro de publication internationale: WO 2017/220426

(56) Documents cités:
- EP-A1- 2 722 075
- FR-A1- 3 031 040
- US-A1- 2006 134 059
- US-A1- 2015 164 813

## Description

La présente invention a pour objet un nouvel extrait de plante, le procédé pour sa préparation ainsi que son utilisation comme agent actif cosmétique, et les compositions cosmétiques, pharmaceutiques, dermo-pharmaceutiques à usage topique en comprenant.

La peau humaine constitue la première image offerte au regard d'autrui et par conséquent, l'amélioration de son aspect est un sujet de préoccupation constant pour les êtres humains. La peau est le reflet d'un état de bien-être, souvent associé à la jeunesse, et a *contrario* à un état de fatigue et/ou de vieillissement. Le vieillissement cutané est donc une préoccupation pour les humains et plus particulièrement pour les consommateurs de produits cosmétiques qui recherchent des solutions pour atténuer et/ou pour prévenir les manifestations visibles liées à cevieillissement. Ce dernier s'observe au niveau des différents tissus cutanés et se caractérise par des altérations métaboliques, fonctionnelles, cellulaires, architecturales et tissulaires, conduisant à des effets externes visibles caractérisés par l'apparition et l'accroissement des rides, par un teint terne, par un manque d'uniformité du teint (phénomène de dyschromie), ou encore par une modification de la texture et des propriétés, notamment biomécaniques, de la peau du corps humain.

Le vieillissement cutané résulte d'une part de facteurs propres à chaque individu (caractéristiques du patrimoine génétique propre à chaque individu) et d'autre part de facteurs environnementaux. Parmi ceux qui peuvent provoquer le vieillissement cutané, on peut citer l'exposition répétée et prolongée au soleil, et plus particulièrement l'exposition aux rayonnements ultra-violets, l'exposition à la pollution atmosphérique, à la fumée de cigarette, les stress oxydants divers pouvant résulter entre autres des facteurs précédemment cités, ainsi que les stress psychologiques, émotionnels et nerveux. L'exposition répétée et prolongée de la peau humaine aux rayonnements du soleil, et plus particulièrement aux rayonnements ultra-violets, conduit à une forme de vieillissement que l'on nomme communément le photo-vieillissement. Ce photo-vieillissement est bien documenté dans la littérature scientifique ; il provoque des altérations de la peau à différents niveaux dont celle, la plus connue, est l'élastose solaire qui se caractérise par des modifications profondes dans l'architecture et l'organisation des fibres élastiques du derme.

Le derme est le principal tissu cutané de structure de la peau. Il s'agit d'un tissu conjonctif dense peu vascularisé. Il comporte des fibroblastes qui sont les principales cellules à l'origine de la synthèse et de l'entretien de ce tissu. Ces cellules baignent dans une matrice extracellulaire qui est composée principalement de fibres de collagène, de fibres élastiques, de glycosaminoglycanes tels que l'acide hyaluronique, de protéoglycannes et de glycoprotéines de structure telle que la fibronectine. Afin d'assurer l'entretien et le renouvellement de ce tissu, les fibroblastes synthétisent en permanence ces macromolécules ainsi que des enzymes spécifiques permettant leur dégradation. Parmi ces enzymes, on peut citer les élastases, enzyme dégradant spécifiquement les fibres élastiques, et les métalloprotéases matricielles (MMP), enzymes dégradant les macromolécules matricielles telles que les collagènes, les protéoglycannes et la fibronectine. Ces enzymes voient leur niveau d'expression augmenter avec le vieillissement (chronologique photo/induit, ou lié aux conditions environnementales) ; elles favorisent la déstructuration du derme qui se traduit par la formation de rides et leur accentuation au cours du temps. La classe des MMP regroupe différentes enzymes dont les collagènases (MMP-1, MMP-8 et MMP-13), capables de dégrader les collagènes fibrillaires à triple hélice. [G.J. Fisher et al. "Molecular mechanisms of photoaging and its prévention by retinoic acid: ultraviolet irradiation induces MAP kinase signal transduction cascades that induce Ap-1-regulated matrix metalloproteinases that dégradé humanskin in vivo."J. Investig. Dermatol. Symp. Proc. 1998 Aug; 3(1):61-8.]

Les modifications du derme conduisent à un aspect caractéristique des peaux altérées par le photovieillissement et qui présentent des rides très profondes et marquées, induisant un aspect de peau tannée, à savoir raide, craquelée et brunie, ainsi que des modifications de leurs propriétés mécaniques. Ces dernières sont dues à l'altération de la matrice extracellulaire dermique, composée par les fibres élastiques et les fibres de collagène, et également à l'altération des caractéristiques cellulaires. Schulze et al. ont montré que les fibroblastes dermiques se rigidifiaient avec l'âge, influençant des fonctions cellulaires impliquant le cytosquelette, telles que les propriétés contractiles, migratoires et prolifératives, qui sont importantes pour la réorganisation de la matrice extracellulaire (Schulze et al., "Stiffening of human skin fibroblasts with age"; Clin. Plast. Surg.; 2012 ; 39(1):9-20).

Les espèces réactives de l'oxygène (connues sous le nom de « ROS ») en excès dans la peau humaine (et ce, que le stimulus soit exogène ou que la production soit endogène) créent des liaisons irréversibles avec les protéines, identifiées sous le terme « protéines carbonylées », qui perdent alors leur fonction. Un lien a récemment été mis en évidence entre ces protéines carbonylées et leur impact sur des fonctions clés cellulaires telles que le métabolisme des carbohydrates, l'entretien des protéines, la mobilité cellulaire, incluant la migration et l'homéostasie protéique [Baraibar et Friguet, "Oxidative proteome modifications target spécifie cellular pathways during oxidative stress, cellular senescence and ageing"; Exp. Gerontol. 2013; 48(7):620-5].

Des études investiguant l'effet de sérums humains, provenant de donneurs de différents âges, sur les propriétés migratoires des fibroblastes ont été conduites par Kondo et al. [Kondo et al. "Inhibitory effects of human sérum on human fetal skin fibroblast migration: migration-inhibitory activity and substances in sérum, and its age-related changes"; In Vitro Cell Dev. Biol. Anim.; 2000; 36(4):256-61]. Les données obtenues montrent que le sérum de donneurs âgés inhibe les propriétés migratoires des fibroblastes, et même celles de fibroblastes foetaux. Ceci illustre l'importance des facteurs intrinsèques dans la problématique du vieillissement cutané.

La technique dite «photodynamique » a été décrite comme étant particulièrement adaptée pour le rajeunissement (exemple : la réduction des rides et ridules, des tâches pigmentaires, ...) des peaux dites « photo-exposées », à savoir les peaux exposées aux rayonnements solaires, et plus particulièrement aux rayonnements ultra-violets. Le mécanisme par lequel cette technique agit a récemment été étudié et il s'avère que son mode d'action passe notamment par une augmentation de la population fibroblastique ainsi que par une augmentation de la capacité migratoire desdits fibroblastes [Jang et al., "Prolonged activation of ERK contributes to the photorejuvenation effect in photodynamis therapy in human dermal fibroblasts"; JID ; 2013 ; 133(9):2265-75].

Ces récentes études montrent que l'altération des propriétés migratoires des fibroblastes contribue au phénomène de vieillissement cutané. Ces propriétés migratoires sont importantes et décrites dans le cadre du processus de réparation des lésions cutanées. Des dysfonctionnements de ce processus chez les personnes âgées sont largement décrits, illustrant l'importance de cette fonctionnalité cellulaire.

Il en résulte qu'une amélioration des propriétés migratoires des fibroblastes du derme de la peau humaine et/ou qu'une augmentation de la population fibroblastique constituent des moyens de prévenir et/ou traiter le vieillissement de le peau du corps humain, et plus particulièrement de prévenir et/ou de traiter les effets visibles dudit vieillissement par exemple les rides, le teint terne, le manque d'uniformité du teint (dyschromie), la rigidité de la peau, causé par le vieillissement naturel ou par une exposition prolongée au soleil, et plus particulièrement à une exposition aux rayonnements ultra-violets, ou par une exposition à des stress oxydants.

Des procédés physiques destinés à stimuler la migration des fibroblastes de la peau humaine sont connus, et parmi ceux-ci, on peut citer l'irradiation au laser de faible intensité (exposition à une longueur d'onde de 632,2 nm) plus particulièrement destinée aux patients atteints de diabète [Houreld et al. « Low-intensity laser irradiation stimulates wound healing in diabetic wounded fibroblast cells (WS1) », 2010, Diabètes Technol Ther, Dec; 12(12*)].*

Il existe également de nombreux principes actifs pharmaceutiques qui stimulent la migration des fibroblastes, et qui sont principalement prescrits dans le cadre d'un processus de cicatrisation. On peut ainsi citer la lactoferrine humaine recombinante produite par des plans de riz transgéniques [Tang et al., « A rice-derived recombinant human lactoferrin stimulates fibroblast prolifération, migration, and sustains cell survival », 2010, Wound Repair Regen, Jan-Feb 18(1)]. Ces procédés physiques et ces principes actifs pharmaceutiques sont généralement mis en oeuvre pour des individus atteints d'une pathologie impactant la qualité de leur peau, et ne sont pas adaptés pour des utilisations cosmétiques.

Il existe également des principes actifs cosmétiques qui sont des extraits de plantes ou de bactéries, et qui sont décrits comme agissant sur la migration des fibroblastes. On peut ainsi citer les extraits de curcuma, qui stimulent la cicatrisation lorsqu'ils sont utilisés à faibles doses et qui agissent sur la migration des fibroblastes à plus forte dose [Demirovic et al., « Curcumin induces stress response and hormetically modulates wound healing ability of human skin fibroblasts undergoing ageing in vitro », 2011, Biogerontology, Mar 6*]* ; l'huile extraite de noix de *Pouteria lucuma*, se caractérisant par la présence majoritaire d'acides linoléique, oléique, palmitique, stéarique et γ-linolénique, et décrite comme stimulant la migration des fibroblastes et l'expression de la vinculine, et adaptées à l'accélération de la cicatrisation des plaies cutanées [Rojo et al., « Wound healing properties of nut oil from Pouteria lucuma », 2010, J. Cosmet. Dermatol., Sep 9(3)]. ;

La combinaison d'extraits de *Vigna arina*, *Cocos nucifera* ., *Terminalia atappa* et *Hibiscus tiliaceus* présente dans des compositions cosmétiques pour permettre de traiter les plaies, d'améliorer la cicatrisation, de traiter les problèmes cutanés liés à l'âge, est décrite dans la demande internationale publiée sous le numéro WO 2010/127396 A1.

La demande internationale publiée sous le numéro WO 2010 /056908 A1 divulgue l'utilisation d'extrait de *Pouteria lucuma* et plus particulièrement de l'huile contenue dans leur noyau, pour améliorer la migration des fibroblastes humains. Cependant, l'utilisation d'extraits de plantes et/ou de bactéries présente comme inconvénient de montrer des performances non fiables dans le temps du fait de la variabilité du contenu des matières premières.

Il existe également des principes actifs cosmétiques de synthèse par exemple des peptides qui sont décrits comme agissant sur la migration des fibroblastes. On peut ainsi citer des pentapeptides de formule Lys-Thr-Thr-Lys-X où X représente un quelconque acide aminé naturel mais préférentiellement la sérine et dont une chaîne d'acides gras (C2 à C22) est greffée sur l'amine N-terminale et/ou son groupe carboxyle estérifié, décrits dans la demande de brevet français publiée sous le numéro FR 2 783 169. Ces pentapeptides sont incorporés dans des compositions cosmétiques ou pharmaceutiques pour provoquer l'augmentation de la synthèse de collagène et de glycosaminoglycanes (démontrée par radioactivité) sur des explants cutanés et l'augmentation de la prolifération de fibroblastes humains normaux en culture, et par conséquent pour améliorer l'aspect de la peau (dans le cas de son vieillissement naturel), de son dessèchement et de sa cicatrisation.

Le document US 2015/164813 divulgue des extraits de Plumbago auriculata obtenus par un procédé comprenant une étape de macération dans une solution hydro-alcoolique (95% éthanol), suivi de plusieurs étapes d'extraction liquide-liquide par mise en contact de la phase aqueuse avec des solvants organiques de caractéristiques différentes ; premièrement un solvant apolaire, deuxièmement un solvant présentant une certaine polarité (le chloroforme), et finalement un solvant polaire et protique (le 1-butanol).

La demande internationale publiée sous le numéro WO 97/17835 divulgue des peptides contenant au moins une séquence de trois acides aminés (Lys-Lys-Gly, Gly-His-Lys ou Glu-His-Lys) conjugués à un acide mono- ou dicarboxylique, incorporés dans des compositions cosmétiques ou pharmaceutiques comme agent cicatrisant et antirides, montrant un effet sur la synthèse de collagène I par les fibroblastes.

*"Armeria maritima"* ou gazon d'Olympe est une plante vivace, gazonnante, de 5 à 30 cm de hauteur. Ses feuilles sont linéaires-obtuses planes et possèdent une seule nervure. Les hampes sont légèrement pubescentes. La gaine d'environ 10 cm est généralement plus courte que le capitule, celui-ci est dense et large (environ 15cm de largeur). Les fleurs sont rosées, rarement blanches. L'involucre est vert ferrugineux, à folioles sur 3 ou 4 rangs, les extérieures ovales, acuminées ou mucronnées, herbacées sur le dos, scarieuses sur les bords. Le calice est à tube, égalant le pédicelle, à côtes aussi larges que les sillons, à lobes ovales, atténués en arête beaucoup plus courte qu'eux. En France, cette plante croit sur les pelouses et falaises du littoral de l'Océan Atlantique et de la Manche. On la retrouve également en Europe occidentale et boréale ainsi qu'en Asie boréale. A noter également que cette plante est souvent utilisée comme plante ornementale pour fleurir des parterres.

Le genre *Armeria* appartient à l'ordre des Caryophyllales et plus particulièrement à la famille des Plumbaginacées. *Armeria maritima* est une plante halophile, c'est-à-dire capable de croître dans un environnement salin, tout comme d'autres espèces de la famille des Plumbaginacées comme celles appartenant augenre *Limonium.* Pour résister au stress salin, les halophytes peuvent synthétiser des métabolites osmoprotecteurs comme par exemple la glycine-bétaïne mais aussi des métabolites permettant de protéger contre le stress oxydatif lié directement au stress salin. [Rathinasabapathi, B.et al. "Osmoprotectant β-alanine betaine synthesis in the Plumbaginaceae: S-adenosyl- I-methionine dépendent N-methylation of β-alanine to its betaine is via N-methyl and N,N-dimethyl β-alanines", Physiol. Plant. 109, 225-231 (2000)].

La demande de brevet européen publiée sous le numéro EP 2 722 075 A1 divulgue l'utilisation d'un extrait aqueux *d'Armeria maritima* obtenu par un procédé mettant en oeuvre du dioxyde de carbone supercritique, pour traiter les irritations et inflammations cutanées.

Cependant, il a été constaté que certains extraits de cette plante riche en composés flavonoïdes étaient phototoxiques.

C'est pourquoi les inventeurs se sont attachés à développer un procédé d'extraction de composés issus de la plante *"Armeria maritima"*, qui permette d'obtenir des extraits non phototoxiques utilisables en cosmétique.

Selon un premier aspect, l'invention a pour objet un procédé pour préparer un extrait butanolique issu d'une biomasse de parties aériennes *d'Armeria maritima*, comprenant les étapes successives suivantes :
- Une étape A₀) de récolte des dites parties aériennes d'*Armeria maritima,* pour en obtenir une récolte des dites parties aériennes ;
- Une étape A₁) de séchage de ladite récolte obtenue à l'étape A₀) ;
- Une étape A₂) de broyage de ladite récolte séchée à l'étape A₁), pour obtenir un broyat de ladite récolte séchée ;
- si nécessaire ou si désiré, une étape A₃) de pulvérisation du broyat obtenu à l'étape A₂), pour obtenir une poudre de ladite récolte séchée ;
- Au moins une étape A) de macération dans une solution hydro-éthanolique de ratio volumique éthanol/eau égal à 80/20, de ladite poudre obtenue à l'étape A₃, pour en obtenir une suspension hydro-éthanolique ;
- Au moins une étape B) de filtration de ladite suspension hydro-éthanolique obtenue à l'étape A), pour en séparer un extrait hydro-éthanolique ;
- Une étape C) de séchage dudit extrait hydro-éthanolique obtenu à l'étape B), pour en obtenir un résidu sec ;
- Une étape D) de solubilisation dans l'eau dudit résidu sec obtenu à l'étape C), pour obtenir une phase aqueuse φ₀ ;
- Au moins une étape E₁) d'extraction liquide-liquide par mise en contact de ladite phase aqueuse φ₀ obtenue à l'étape D), avec du cyclohexane en un ratio volumique cyclohexane/phase aqueuse φ₀ égal à 2/3, pour obtenir après séparation des phases non miscibles, une nouvelle phase aqueuse φ₁ ;
- Au moins une étape E₂ₐ) d'extraction liquide-liquide par mise en contact de ladite phase aqueuse φ₁ obtenue à l'étape E₁), avec du dichlorométhane en un ratio volumique dichlorométhane/phase aqueuse φ₁ égal à 2/3, pour obtenir après séparation des phases non miscibles, une nouvelle phase aqueuse φ₂ₐ;
- Au moins une étape E_{2b}) d'extraction liquide-liquide par mise en contact de ladite phase aqueuse φ₂ₐ obtenue à l'étape E₂ₐ), avec de l'acétate d'éthyle en un ratio volumique acétate d'éthyle/phase aqueuse φ₂ₐ égal à 2/3, pour obtenir après séparation des phases non miscibles, une nouvelle phase aqueuse φ_{2b};
- Au moins une étape E₃) d'extraction liquide-liquide par mise en contact de ladite phase aqueuse φ_{2b} obtenue à l'étape E_{2b}), avec du 1-butanol saturé en eau en un ratio volumique 1-butanol/phase aqueuse φ_{2b}, égal à 2/3, pour obtenir après séparation des phases non miscibles, ledit extrait butanolique attendu et une nouvelle phase aqueuse φ₃.

Par parties aériennes de la plante, on entend les feuilles, tiges et/ou fleurs de ladite plante.

L'étape C) du procédé tel que défini précédemment est généralement effectuée au moyen d'un évaporateur rotatif jusqu'au séchage complet.

Selon l'étape D) du procédé tel que défini ci-dessus, la solubilisation dans l'eau du résidu sec obtenu à l'issue de l'étape C), est de préférence réalisée avec de l'eau déminéralisée jusqu'à la limite de saturation du résidu sec et assistée aux ultra-sons, pour en améliorer le rendement.

L'invention a aussi pour objet un extrait butanolique d'une biomasse de parties aériennes d'*Armeria maritima*, obtenu par le procédé tel que défini ci-dessus.

L'invention a aussi pour objet, l'utilisation de l'extrait butanolique d'une biomasse de parties aériennes d'*Armeria maritima* tel que défini ci-dessus, comme agent actif cosmétique anti-âge.

L'invention aussi a pour objet une composition cosmétique (C₁), à usage topique comprenant au moins un excipient cosmétiquement acceptable et une quantité efficace de l'extrait butanolique tel que défini ci-dessus.

Selon l'invention, ladite composition (C₁) est généralement étalée sur la surface de la peau à traiter, puis la peau est massée quelques instants.

L'expression "à usage topique" utilisée dans la définition de la composition (C₁) objet de la présente invention, signifie que ladite composition (C₁) est mise en oeuvre par application sur la peau, qu'il s'agisse d'une application directe ou d'une application indirecte lorsque ladite composition (C₁) selon l'invention est imprégnée sur un support destiné à être mis en contact avec la peau (papier, lingette, textile, dispositif transdermique, etc....).

L'expression « cosmétiquement acceptable » utilisée dans la définition de la composition (C₁) objet de la présente invention, signifie selon la directive du Conseil de la Communauté Economique Européenne N°76/768/CEE du 27 juillet 1976 modifiée par la directive N°93/35/CEE du 14 juin 1993, qu'elle comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Par « agent actif cosmétique anti-âge », on entend au sens de la présente invention une substance chimique ou une composition chimique ou un extrait issue de biomasse d'une plante dont la propriété ou la fonction technique consiste à empêcher ou à ralentir l'apparition des signes du vieillissement de la peau humaine ou des lèvres ou bien d'éliminer lesdits signes.

Par " signes du vieillissement de la peau humaine ou des lèvres" on entend, au sens de l'invention, toutes modifications de l'aspect extérieur de la peau ou des lèvres dues au vieillissement, qu'elles soient chronobiologiques et/ou photo-induites et/ou résultant de l'exposition à des stresses environnementaux (pollution atmosphérique, contact avec substances dangereuses), comme les rides et ridules, l'altération du microrelief, le manque d'élasticité et/ou de tonus de la peau, le manque de densité et/ou de fermeté de la peau humaine ou des lèvres, mais également toutes modifications internes de la peau qui ne se traduisent pas systématiquement par un aspect extérieur modifié, comme par exemple toutes dégradations internes de la peau consécutives à une exposition aux rayonnements ultra-violets.

Par quantité efficace de l'extrait butanolique issu d'une biomasse de parties aériennes d'Armeria maritima tel que défini précédemment, on entend pour 100% de la masse de ladite composition (C₁), la quantité comprise entre 0,1% et 5% massique, plus particulièrement entre 0,1% et 3% massique, et encore plus particulièrement entre 0,5% et 2 % massique dudit extrait butanolique issu d'une biomasse de parties aériennes d'Armeria maritima.

La composition (C₁) objet de la présente invention, se présente généralement sous forme d'une solution aqueuse ou hydro-alcoolique ou hydro-glycolique, sous forme d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile, ou sous forme d'une poudre.

La composition (C₁) objet de la présente invention, peut être conditionnée dans un flacon, dans un dispositif de type "flacon" pompe, sous forme pressurisée dans un dispositif aérosol, dans un dispositif muni d'une paroi ajourée comme une grille ou dans un dispositif muni d'un applicateur à billes (dit "roll-on").

De façon générale, l'extrait de l'extrait butanolique issu d'une biomasse de parties aériennes d'*Armeria maritima* objet de la présente invention, est associé à des additifs chimiques habituellement mis en oeuvre dans le domaine des formulations à usage topique, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les eaux thermales ou minérales les agents plastifiants, les agents émulsionnants et co-émulsionnants, les agents opacificants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents que l'on peut associer à l'extrait butanolique issu d'une biomasse de parties aériennes *d'Armeria maritima* dans la composition (C₁), on peut citer les tensioactifs moussants et/ou détergents anioniques, cationiques, amphotères ou non ioniques.

Parmi les tensioactifs anioniques moussants et/ou détergents, on peut citer les sels de métaux alcalins, de métaux alcalino-terreux, d'ammonium, d'amines, ou d'aminoalcools d'alkylether sulfates, d'alkyl sulfates, d'alkylamidoéther sulfates, d'alkylaryl polyéthersulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkyl sulfosuccinates, d'alkyléther sulfosuccinates, d'alkylamide sulfosuccinates, d'alkyl sulfoacétates, d'alkyl sarcosinates, d'acyl iséthionates, de N-acyl taurates, d'acyl lactylates, de dérivés N-acylés d'acides aminés, de dérivés N-acylés de peptides, de dérivés N-acylés de protéines, de dérivés N-acylés d'acides gras.

Parmi les tensioactifs amphotères moussants et/ou détergents, on peut citer les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents, on peut citer particulièrement les dérivés d'ammoniums quaternaires.

Parmi les tensioactifs non ioniques moussants et/ou détergents, on peut citer plus particulièrement les alkylpolyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer à l'extrait butanolique issu d'une biomasse de parties aériennes d'*Armeria maritima* dans la composition (C₁), on peut citer les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE^{™} LT et GLUMATE^{™} DOE120 ; les esters gras alcoxylés tels que le PEG 150 pentaérythrytyl tétrastéarate commercialisé sous l'appellation CROTHIX^{™} DS53, le PEG 55 propylène glycol oléate commercialisé sous l'appellation ANTIL^{™} 141 ; les carbamates de polyalkylène glycols à chaînes grasses comme le PPG-14 laureth isophoryl dicarbamate commercialisé sous l'appellation ELFACOS^{™} T211, le PPG-14 palmeth-60 hexyl dicarbamate commercialisé sous l'appellation ELFACOS^{™} GT2125.

Comme exemples d'agents épaississants et/ou gélifiants que l'on peut associer à l'extrait butanolique d'une biomasse de parties aériennes d'*Armeria maritima* dans la composition (C₁), on peut citer les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide 2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- methyl)acrylamido méthane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (VIII) :

CH₂=C(R'₃)-C(=O)-[CH₂-CH₂-O]ₙ-R'₄ (VIII)

dans laquelle R'₃ représente un atome d'hydrogène ou un radical méthyle, R'₄ représente un radical alkyle linéaire ou ramifié comportant de huit à trente atomes de carbone et n représente un nombre supérieur ou égal à un et inférieur ou égal à cinquante.

Les polymères de type polyélectrolytes, linéaires ou branchés ou réticulés que l'on peut associer à l'extrait butanolique issu d'une biomasse de parties aériennes d'*Armeria maritima* dans la composition (C₁), peuvent se présenter sous la forme d'une solution, d'une suspension aqueuse, d'une émulsion eau-dans-huile, d'une émulsion huile-dans-eau, d'une poudre. Les polymères de type polyélectrolytes, linéaires ou branchés ou réticulés que l'on peut associer à l'extrait butanolique issu d'une biomasse de parties aériennes d'*Armeria maritima* dans la composition (C₁), peuvent être sélectionnés parmi les produits commercialisés sous les appellations SIMULGEL^{™} EG, SIMULGEL^{™}EPG, SEPIGEL^{™} 305, SIMULGEL^{™} 600, SIMULGEL^{™} NS, SIMULGEL^{™} INS 100, SIMULGEL^{™} FL, SIMULGEL^{™} A, SIMULGEL^{™} SMS 88, SEPINOV^{™}EMT 10, SEPIPLUS^{™}400, SEPIPLUS^{™}265, SEPIPLUS^{™}S, SEPIMAX^{™}Zen, ARISTOFLEX^{™}AVC, ARISTOFLEX^{™}AVS, NOVEMER^{™}EC-1, NOVEMER^{™}EC 2, ARISTOFLEX^{™}HMB, COSMEDIA^{™}SP, FLOCARE^{™}ET 25, FLOCARE^{™}ET 75, FLOCARE^{™}ET 26, FLOCARE^{™}ET 30, FLOCARE^{™}ET 58, FLOCARE^{™}PSD 30, VISCOLAM^{™}AT 64, VISCOLAM^{™}AT 100.

Comme exemples d'agents épaississants et/ou gélifiants que l'on peut associer à l'extrait butanolique issu d'une biomasse de parties aériennes d'*Armeria maritima* dans la composition (C₁), on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ).

Comme exemples d'agents épaississants et/ou gélifiants que l'on peut associer à l'extrait butanolique issu d'une biomasse de parties aériennes d'*Armeria maritima* dans la composition (C₁), on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants que l'on peut associer à l'extrait butanolique issu d'une biomasse de parties aériennes d'*Armeria maritima* dans la composition (C₁), on peut citer la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer à l'extrait butanolique issu d'une biomasse de parties aériennes d'*Armeria maritima* dans la composition (C₁), on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants que l'on peut associer à l'extrait butanolique issu d'une biomasse de parties aériennes d'*Armeria maritima* dans la composition (C₁), on peut citer l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales que l'on peut associer à l'extrait butanolique issu d'une biomasse de parties aériennes d'*Armeria maritima* dans la composition (C₁), on peut citer les eaux thermales ou minérales ayant une minéralisation d'au moins 300 mg/I, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer à l'extrait butanolique issu d'une biomasse de parties aériennes d'*Armeria maritima* dans la composition (C₁), on peut citer les xylènes sulfonates, les cumènes sulfonates, l'hexyl polyglucoside, le (2-éthyl hexyl) polyglucoside ou le n-heptyl polyglucoside.

Comme exemples d'agents tensioactifs émulsionnants que l'on peut associer à l'extrait butanolique issu d'une biomasse de parties aériennes d'*Armeria maritima* dans la composition (C₁), on peut citer les tensioactifs non ioniques, des tensioactifs anioniques, des tensioactifs cationiques.

Comme exemples de tensioactifs non-ioniques émulsionnants que l'on peut associer à l'extrait butanolique issu d'une biomasse de parties aériennes d'*Armeria maritima* dans la composition (C₁), on peut citer les esters d'acides gras et de sorbitol, comme les produits commercialisés sous les appellations MONTANE^{™}40, MONTANE^{™}60, MONTANE^{™}70, MONTANE^{™}80 et MONTANE^{™}85 ; les compositions comprenant du stéarate de glycérol et l'acide stéarique éthoxylé entre cinq moles et cent cinquante moles d'oxyde d'éthylène, comme la composition comprenant de l'acide stéarique éthoxylé à cent trente-cinq moles d'oxyde d'éthylène et du stéarate de glycérol commercialisée sous l'appellation SIMULSOL^{™} 165 ; les esters de mannitan ; les esters de mannitan éthoxylés ; les esters de sucrose ; les esters de méthyl glucoside ; les alkyl polyglycosides comportant un radical aliphatique, linéaire ou ramifié, saturé ou insaturé, et comportant de quatorze à trente-six atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le (2-octyl dodécyl) polyxyloside, le (12-hydroxy stéaryl) polyglucoside ; les compositions d'alcools gras linéaires ou ramifiés, saturés ou insaturés, et comportant de quatorze à trente-six atomes de carbone, et d'alkyl polyglycosides tels que décrits précédemment, par exemple les compositions commercialisées sous les noms de marque MONTANOV^{™}68, MONTANOV^{™}14, MONTANOV^{™}82, MONTANOV^{™}202, MONTANOV^{™}S, MONTANOV^{™}WO18, MONTANOV^{™}L, FLUIDANOV^{™}20X et EASYNOV^{™}.

Comme exemples de tensioactifs anioniques que l'on peut associer à l'extrait butanolique issu d'une biomasse de parties aériennes d'*Armeria maritima* dans la composition (C₁), on peut citer le glycéryl stéarate citrate, le cétéarylsulfate, les savons comme le stéarate de sodium ou le stéarate de triéthanolammonium, les dérivés N-acylés d'acides aminés salifiés comme par exemple le stéaroyl glutamate.

Comme exemples de tensioactifs cationiques émulsionnants que l'on peut associer à l'extrait butanolique issu d'une biomasse de parties aériennes d'*Armeria maritima* dans la composition (C₁), on peut citer les aminoxydes, le quaternium-82 et les tensioactifs décrits dans la demande de brevet WO 96/00719 et principalement ceux dont la chaîne grasse comprend au moins seize atomes de carbone.

Comme exemples d'agents opacifiants et/ou nacrants que l'on peut associer à l'extrait butanolique issu d'une biomasse de parties aériennes d'*Armeria maritima* dans la composition (C₁), on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de douze à vingt-deux atomes de carbone.

Comme exemples d'agents de texture que l'on peut associer à l'extrait butanolique issu d'une biomasse de parties aériennes d'*Armeria maritima* dans la composition (C₁), on peut citer des dérivés N-acylés d'acides aminés, comme la lauroyl lysine commercialisée sous l'appellation AMINOHOPE^{™}LL, l'octenyl starch succinate commercialisé sous l'appellation DRYFLO^{™}, le myristyl polyglucoside commercialisé sous l'appellation MONTANOV^{™} 14, les fibres de cellulose, les fibres de coton, les fibres de chitosane, le talc, la séricite, le mica.

Comme exemples d'agents déodorants que l'on peut associer à l'extrait butanolique issu d'une biomasse de parties aériennes d'*Armeria maritima* dans la composition (C₁), on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2-décanediol ; le 1,3-propanediol ; l'acide salicylique ; le bicarbonate de sodium ; les cyclodextrines ; les zéolithes métalliques ; le TRICLOSAN^{™} ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'huiles que l'on peut associer à l'extrait butanolique issu d'une biomasse de parties aériennes d'*Armeria maritima* dans la composition (C₁), on peut citer les huiles minérales telles que l'huile de paraffine, l'huile de vaseline, les isoparaffines ou les huiles blanches minérales ; les huiles d'origine animale, telles que le squalène ou le squalane ;les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer à l'extrait butanolique issu d'une biomasse de parties aériennes d'*Armeria maritima* dans la composition (C₁), on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs que l'on peut associer à l'extrait butanolique issu d'une biomasse de parties aériennes d'*Armeria maritima* dans la composition (C₁), on peut citer les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le ω-undecylènoyl phénylalanine commercialisé sous l'appellation SEPIWHITE^{™}MSH, le SEPICALM^{™}VG, le mono ester et/ou le diester de glycérol du w-undecylènoyl phénylalanine, les w-undecylènoyl dipeptides, l'arbutine, l'acide kojique, l'hydroquinone ; les composés montrant une action apaisante notamment le SEPICALM^{™} S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylglucoside, la composition commercialisée sous le nom de marque AQUAXYL^{™}, la composition commercialisés sous le nom de marque PRO-XYLANE^{™}, les dérivés de C-glycosides et plus particulièrement les dérivés de C-glucosides, de C-xylosides ; les extraits végétaux riches en polyphénols comme les extraits de raisin, les extraits de pin, les extraits de vin, les extraits d'olive ; les composés montrant une action amincissante ou lipolytique comme la caféine ou ses dérivés, l'ADIPOSLIM^{™}, l'ADIPOLESS^{™}, la fucoxanthine ; les protéines N-acylées ; les peptides N-acylés comme le MATRIXIL^{™} ; les acides aminés N-acylés ; les hydrolysâts partiels de protéines N-acylés ; les acides aminés ; les peptides ; les hydrolysâts totaux de protéines ; les extraits de soja, par exemple la Raffermine^{™} ; les extraits de blé par exemple la TENSINE^{™} ou la GLIADINE^{™} ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE^{™} C8G, le LIPACIDE^{™} UG, le SEPICONTROL^{™} A5, le FLUIDIPURE^{™}8G ; l'OCTOPIROX^{™} ou le SENSIVA^{™} SC50 ; les composés montrant une propriété énergisante ou stimulante comme le PHYSIOGENYL^{™}, le panthénol et ses dérivés comme le SEPICAP^{™} MP ; les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE^{™} ; le SURVICODE^{™} ; les actifs anti-photo vieillissement ; les actifs protecteurs de l'intégrité de la jonction dermoépidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica*, de fucus, de romarin, de saule ; les agents de bronzage ou de brunissement de la peau, par exemple la dihydroxyacétone (DHA), l'érythrulose, l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, par exemple les caroténoïdes ( et plus particulièrement le beta carotène et le gamma carotène), le produit commercialisé sous le nom de marque « Carros oil » (Nom INCI : *Daucus carotta*, *Helianthes annuus* Sunflower oil) par la société Provital, qui contient des caroténoïdes, de la vitamine E et de la vitamine K ; la tyrosine et/ou ses dérivés, connus pour leur effet sur l'accélération du bronzage de la peau humaine en association avec une exposition aux rayonnements ultra-violets, par exemple le produit commercialisé sous le nom de marque « SunTan Accelerator^{™} » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze^{™} (nom INCI : Acetyl Tyrosine, Monk's pepper extract (*Vitex Agnus-castus*)) par la société Mibelle qui contient de l'acétyl tyrosine, produit commercialisé sous le nom de marque Unipertan VEG-24/242/2002 (nom INCI : butylène glycol and Acetyl Tyrosine and hydrolyzed vegetable protein and Adenosine triphosphate) par la société UNIPEX, le produit commercialisé sous le nom de marque « Try-Excell^{™} » (nom INCI : Oleoyl Tyrosine and *Luffa Cylindrica* (Seed) Oil and Oleic acid) par la société Sederma qui contient des extraits de pépins de courge (ou huile de Loofah), le produit commercialisé sous le nom de marque «Actibronze^{™} » (nom INCI : hydrolyzed wheat protein and acetyl tyrosine and copper gluconate) par la société Alban Muller, le produit commercialisé sous le nom de marque Tyrostan^{™} (nom INCI : potassium caproyl tyrosine) par la société Synerga, le produit commercialisé sous le nom de marque Tyrosinol (nom INCI : Sorbitan Isostearate, glyceryl oleate, caproyl Tyrosine) par la société Synerga, le produit commercialisé sous le nom de marque InstaBronze^{™} (nom INCI : Dihydroxyacétone and acetyl tyrosine and copper gluconate) commercialisé par la société Alban Muller, le produit commercialisé sous le nom de marque Tyrosilane (nom INCI : méthylsilanol and acétyl tyrosine) par la société Exymol ; les peptides connus pour leur effet d'activation de la mélanogénèse par exemple le produit commercialisé sous le nom de marque Bronzing SF Peptide powder (nom INCI : Dextran and Octapeptide-5) par la société Infinitec Activos, le produit commercialisé sous le nom de marque Melitane (nom INCI : Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions^{™} (nom INCI : Butylène glycol , Palmitoyl Tripeptide-40) par la société LIPOTEC, les sucres et les dérivés de sucres par exemple le produit commercialisé sous le nom de marque Tanositol^{™} (nom INCI : inositol) par la société Provital, le produit commercialisé sous le nom de marque Thalitan^{™} (ou Phycosaccharide^{™} AG) par la société CODIF international (nom INCI : Aqua and hydrolyzed algin (*Laminaria digitata*) and magnésium sulfate and manganèse sulfate) contenant un oligosaccharide d'origine marine (acide guluronique et acide mannuronique chélatés avec les ions magnésium et manganèse), le produit commercialisé sous le nom de marque Melactiva^{™} (nom INCI : Maltodextrin, Mucuna Pruriens Seed extract) par la société Alban Muller, les composés riches en flavonoïdes par exemple le produit commercialisé sous le nom de marque « Biotanning » (nom INCI : Hydrolyzed *Citrus aurantium dulcis* fruit extract) par la société Silab et connu pour être riche en flavonoides de citron (de type hespéridines) ; les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis parmi ceux décrits dans la demande de brevet européen publiée sous le numéro EP 1 515 688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, , des associations de mono- et diester d'acide cinnamique et de vitamine C, et plus généralement ceux cités dans la demande de brevet européen publiée sous le numéro EP 1 515 688 A2.

Comme exemples d'agents antioxydants que l'on peut associer à l'extrait butanolique issu d'une biomasse de parties aériennes *d'Armeria maritima* dans la composition (C₁), on peut citer l'EDTA et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE^{™} GL 47S commercialisé par la société Akzo Nobel sous le nom INCI : Tetrasodium Glutamate Diacetate.

Comme exemples de filtres solaires que l'on peut associer à l'extrait butanolique issu d'une biomasse de parties aériennes d'*Armeria maritima* dans la composition (C₁), on peut citer tous ceux figurant dans la directive cosmétique 76/768/CEE modifiée annexe VII.

Parmi les filtres organiques solaires que l'on peut associer à l'extrait butanolique issu d'une biomasse de parties aériennes d'*Armeria maritima* dans la composition (C₁), on peut citer la famille des dérivés de l'acide benzoïque comme les acides *para-*aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de (p-isopropanol phényle) ; la famille des dérivés de l'acide cinnamique comme le cinnamate d'éthylhexyle, le cinnamate d'éthyl-4-isopropyle, le cinnamate de méthyl-2,5-diisopropyle, le *p*-méthoxy cinnamate de propyle, le *p*-méthoxy cinnamate d'isopropyle, le *p*-méthoxy cinnamate d'isoamyle, le *p*-méthoxy cinnamate d'octyle, le *p*-méthoxy cinnamate de (2-éthyl hexyle), le *p*-méthoxy cinnamate de (2-éthoxy éthyle), le *p*-méthoxy cinnamate de cyclohexyle, le cinnamate d'éthyl-α-cyano-β-phényle, le cinnamate de (2-éthyl hexyl)-α-cyano-β-phényle, le cinnamate de diparaméthoxy mono-2-éthylhexanoyl de glycéryle ; la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le (2-éthyl hexyl) 4'-phényl benzophénone-2-carboxylate, la 2-hydroxy 4-(n-octyloxy) benzophénone, la 4-hydroxy 3-carboxy benzophénone ; le 3-(4'-méthyl benzylidène) d,l-camphre, le 3-(benzylidène) d,l-camphre, le benzalkonium méthosulfate camphre ; l'acide urocanique, l'urocanate d'éthyle ; la famille des dérivés de l'acide sulfonique comme l'acide sulfonique 2-phényl benzimidazole-5 et ses sels ; la famille des dérivés de la triazine comme l'hydroxyphényl triazine, l'(éthylhexyloxy) (hydroxyphényl) (4-méthoxy phényl) triazine, le 2,4,6-trianillino-(p-carbo-2'-éthylhexyl-1'-oxy)-1,3,5-triazine, le 4,4-((6-(((1,1-diméthyl éthyl) amino) carbonyl) phenyl) amino)-1,3,5-triazine-2,4-diyl diimino) bis(2-éthyl hexyl) ester de l'acide benzoïque, le 2-phényl-5-méthyl benzoxazole, le 2-(2'-hydroxy 5'-méthyl phényl) benzotriazole, le 2-(2'-hydroxy 5'-t-octyl phényl) benzotriazole, le 2-(2'-hydroxy 5'-méthy phényl) benzotriazole; la dibenzazine; le dianisoylméthane, le 4-méthoxy 4"-t-butyl benzoyl méthane ; la 5-(3,3-diméthyl 2-norbornylidène)-3-pentan-2-one ; la famille des dérivés du diphénylacrylate comme le (2-éthyl hexyl) 2-cyano 3,3-diphényl 2-propènoate, l'éthyl-2-cyano 3,3-diphényl 2-propènoate ; la famille des polysiloxanes comme le malonate de benzylidène siloxane.

Parmi les filtres inorganiques solaires, également appelés "écrans minéraux", que l'on peut associer à l'extrait butanolique issu d'une biomasse de parties aériennes d'*Armeria maritima* dans la composition (C₁), on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Les exemples suivants illustrent l'invention, sans toutefois la limiter.

### Préparation d'un extrait butanolique d'Armeria maritima (Extrait AmB)

On prépare l'extrait butanolique d'*Armeria maritima* en mettant en oeuvre les étapes successives suivantes :
1) - Récolte
   Les parties aériennes de la plante en fleur sont récoltées.
2) - Séchage
   La récolte est séchée afin d'éviter sa dégradation et de faciliter sa conservation lors du transport.
3) - Broyage
   Les parties aériennes (tiges, feuilles, fleurs) sont broyées finement afin d'augmenter le rendement d'extraction
4) - Macération hydro-éthanolique
   Un mélange plante/ solvant (éthanol/eau 80/20) est soumis à agitation à l'aide d'une pale. L'extrait est filtré et le marc réextrait (et cela deux fois de suite) afin d'épuiser la matière végétale et augmenter ainsi le rendement d'extraction. Les trois extraits hydro-alcooliques sont assemblés. (**Extrait AmE**)
5) - Séchage de l'extrait hydro-éthanolique
   L'extrait hydro-alcoolique précédemment obtenu est évaporé à sec à l'aide d'un évaporateur rotatif.
6) - Solubilisation maximale de l'extrait dans de l'eau.
   L'extrait est repris dans de l'eau déminéralisée. La solubilisation n'étant pas aisée, le mélange extrait/eau est passé aux ultra-sons afin de faciliter la solubilisation.
7) - Extraction liquide/liquide (phase aqueuse contenant l'extrait mise en contact successivement avec des solvants organiques non miscibles). Pour chaque solvant organique, le volume correspond à 2/3 de celui de la phase aqueuse (Vaq). Néanmoins, pour chaque solvant organique, l'extraction est réalisée trois fois, et on récupère donc 2 Vaq pour chaque solvant organique. Les solvants utilisés sont les suivants:
   - Cyclohexane (**Extrait AmC**)
   - Dichlorométhane (**Extrait AmD**)
   - Acétate d'éthyle (**Extrait AmA**)
   - 1-butanol préalablement saturé en eau (extraiyant les flavonoïdes diglycosides d'intérêt) (**Extrait AmB**)

### Caractérisation de l'Extrait AmB selon l'invention

### Analyse phytochimique de l'extrait AmB

Afin de pouvoir identifier les composés majoritairement présents dans l'extrait butanolique AmB selon l'invention, celui est fractionné par chromatographie liquide sous vide (Phase stationnaire: Silice greffée C18; phase mobile: Solution hydrométhanolique en augmentant progressivement la proportion en méthanol);

Pour certaines des sous-fractions obtenues celles-ci sont de nouveau fractionnées par chromatographie d'exclusion sur colonne Sephadex^{™} LH 20

Pour l'ensemble des sous-fractions, celles-ci sont de nouveau fractionnées par chromatographie semi-préparative en phase inverse.

Les molécules majoritaires purifiées sont analysées par spectrométrie UV, spectrométrie de masse haute résolution (LC-ESI-QTOF) et résonance magnétique nucléaire (RMN 1 et 2D)

Ces analyses permettent d'identifier les composés suivants:

| 3-*O*-α-L-arabinopyranosyl(1->6)β-D-glucopyranosyl-myricétine (Masse molaire: 612g/mol); Formule brute: C₂₆H₁₈O₁₇) | |
|---|---|
| Spectre UV (λₘₐₓ) | ¹H NMR (500MHz, Méthanol-d4) δ |
| 204nm | 3,13 (dd, J=12,40, 1,14Hz, 1H), 3,21 (dd, J=10,07, 3,59Hz, 1H), 3,43 (t, J=8,90Hz, 3 H), 3,54 (dd, J=9,00, 7,78Hz, 2 H), 3,63 (dd, J=12,28, 5,95Hz, 2 H), 3,69 (br dd, J=12,44, 2,75Hz, 1H), 3,92 (dd, J=12,09, 1,49Hz, 1H), 4,04 (d, J=7,17Hz, 1H), 5,19 (d, J=7,86Hz, 1H), 6,20 (d, J=2,06Hz, 1H) 6,40 (d, J=2,06Hz, 1H), 7,34 (s, 2 H) |
| 266nm | |
| 359nm | |

| 3-*O*-α-L-arabinofuranosyl(1->6)β-D-glucopyranosyl-myricétine (Masse molaire: 612g/mol; Formule brute: C₂₆H₁₈0₁₇) | |
|---|---|
| Spectre UV (λₘₐₓ) | ¹H NMR (500MHz, Méthanol-d4) δ |
| 207nm | 3,29 (dd, J=9,61, 8,93Hz, 1H), 3,42 (t, J=9,50Hz, 2 H), 3,45 (br dd, J=11,22, 6,18Hz, 2 H), 3,51 (dd, J=9,08, 8,09Hz, 2 H), 3,56 (dd, J=11,94, 5,00Hz, 2 H), 3,66 (br dd, J=11,94, 3,09Hz, 2 H), 3,74 (dd, J=5,84, 3,40Hz, 1H), 3,81 (ddd, J=6,03, 5,11, 3,20Hz, 1H), 3,84 (br dd, J=3,13, 1,53Hz, 1H), 3,85 (dd, J=1 0,91, 1,98Hz, 1H), 4,76 (d, J=0,92Hz, 1H), 5,14 (d, J=7,78Hz, 1H), 6,20 (d, J=1,98Hz, 1H), 6,38 (d, J=1,98Hz, 1H), 7,26 (s, 2 H) |
| 260nm | |
| 357nm | |

| 3-*O*-α-L-rhamnopyranosyl(1->6)β-D-glucopyranosyl-myricétine (Masse molaire: 626g/mol; Formule brute: C₂₇H₃₀O₁₇) | |
|---|---|
| Spectre UV (λₘₐₓ) | ¹H NMR (500MHz, Méthanol-d4) δ |
| 206nm | 1,12 (br d, J=6,18Hz, 3 H), 3,28 (t, J=9,60Hz, 4 H), 3,40 (dd, J=10,76, 5,84Hz, 1H), 3,41 (t, J=9,00Hz, 1H), 3,50 (dd, J=9,04, 8,13Hz, 2 H), 3,55 (br dd, J=9,44, 3,49Hz, 1H), 3,63 (dd, J=3,32, 1,60Hz, 1H), 3,81 (dd, J=10,99, 3,20Hz, 1H), 4,53 (s, 1H) 5,08 (br d, J=7,78Hz, 2 H), 6,21 (d, J=1,95Hz, 1H), 6,40 (d, J=2,06Hz, 1H), 7,30 (d, J=4,01Hz, 2 H) |
| 262nm | |
| 359nm | |

| 3-O-α-L-arabinopyranosyl(1->6)β-D-glucopyranosyl-quercétine (Masse molaire: 596g/mol; Formule brute: C₂₆H₂₈O₁₆) | |
|---|---|
| Spectre UV (λₘₐₓ) | ¹H NMR (500MHz, Méthanol-d4) δ |
| 202nm | 3,14 (dd, J=12,36, 1,44Hz, 1H), 3,20 (dd, J=8,97, 3,45Hz, 1H), 3,34 - 3,38 (m, 1H), 3,34 - 3,38 (m, 1H), 3,42 (t, J=8,80Hz, 1H), 3,51 (dd, J=9,54, 7,78Hz, 1H), 3,61 (dd, J=12,05, 6,15Hz, 1H), 3,65 (d, J=3,30Hz, 1H), 3,69 (dd, J=12,30, 3,14Hz, 1H), 3,91 (dd, J=12,17, 1,76Hz, 1H), 4,05 (d, J=7,03Hz, 1H), 5,19 (d, J=7,65Hz, 1H), 6,21 (d, J=2,01Hz, 1H), 6,41 (d, J=2,01Hz, 1H), 6,88 (d, J=8,28Hz, 1H), 7,69 (dd, J=8,53, 2,38Hz, 1H), 7,71 (d, J=1,88Hz, 1H) |
| 255nm | |
| 354nm | |

| 3-O-α-L-arabinofuranosyl(1->6)β-D-glucopyranosyl-quercétine (Masse molaire: 596g/mol; Formule brute: C₂₆H₂₈O₁₆) | |
|---|---|
| Spectre UV (λₘₐₓ) | ¹H NMR (500MHz, METHANOL-d4) δ |
| 202nm | n.d. |
| 255nm | |
| 359nm | |

| 3-O-α-L-arabinopyranosyl(1->6)β-D-glucopyranosyl-kaempférol (Masse molaire: 580g/mol; Formule brute: C₂₆H₂₈O₁₅) | |
|---|---|
| Spectre UV (λₘₐₓ) | ¹H NMR (500MHz, METHANOL-d4) δ |
| 200nm | 3,17 (dd, J=12,21, 1,53Hz, 1H), 3,19 (dd, J=8,85, 3,66Hz, 1H), 3,41 (br t, J=8,90Hz, 3 H), 3,48 (br dd, J=9,00, 7,78Hz, 1H), 3,61 (dd, J=12,21, 6,10Hz, 1H), 3,63 - 3,66 (m, 1H), 3,69 (br dd, J=12,36, 2,90Hz, 1H), 3,90 (br dd, J=11,90, 1,83Hz, 1H), 4,06 (d, J=7,02Hz, 1H), 5,13 (br d, J=7,63Hz, 2 H), 6,12 (d, J=1,83Hz, 1H), 6,25 - 6,37 (m, 1H), 6,89 (br d, J=8,85Hz, 2 H), 8,10 (d, J=8,85Hz, 1H) |
| 241nm | |
| 268nm | |
| 349nm | |

### Mise en évidence de l'effet anti-âge de l'Extrait AmB selon l'invention

### Choix du modèle

Le modèle choisi pour mettre en évidence l'effet technique de l'Extrait selon l'invention est un modèle d'étude de l'activité de l'enzyme collagénase. Une collagénase purifiée est utilisée et la dégradation de son substrat pro-fluorescent est suivi par fluorimétrie. Ce test permet de mettre en évidence des effets directs sur la fonctionnalité de l'enzyme elle-même.

### Protocole

L'activité anti-collagénase est évaluée à l'aide d'un kit commercialisé (EnzCheck^{™} gélatinase/collagénase Assay Kit, Molecular Probes, référence E-12055).

Il s'agit d'un test fondé sur une évaluation biochimique, acellulaire, à partir d'une collagénose purifiée d'un champignon (*Clostridium Histolyticum*) et d'une gélatine purifiée à partir de la peau porcine, du collagène de type I purifié à partir de peau bovine ou du collagène de type IV purifié à partir de placenta humain. Les substrats (gélatine, collagène I ou collagène IV) sont conjugués à la fluorescéine, La fluorescence est révélée par la digestion du substrat par la MMP et mesurée avec un fluorimètre (Fluoroskan Ascent FL - Labsystem) : excitation : 495nm, émission : 545nm. L'augmentation de la fluorescence est proportionnelle à l'activité de la MMP. Une diminution de cette fluorescence est attendue en présence d'un actif anti-MMP. Le test est validé par l'utilisation d'un inhibiteur anti-MMP de référence fourni dans le kit, la 1,10-phénanthroline, testé à 0,01% et 0,001%.

| **Produits testés** | **Concentration** | **Solubilité** |
|---|---|---|
| *Armeria maritima* (AmE) | 100 et 50 µg/ml | Solution mère à 10mg/ml en DMSO |
| *Armeria maritima* (AmB) | 100 et 50 µg/ml | Solution mère à 10mg/ml en DMSO |

Les produits sont mis sur plaque de 96 puits de la manière suivante :

| | Blanc/témoin | Témoin | Essai à blanc | Produit |
|---|---|---|---|---|
| Extrait à analyser | - | - | 80µL | 80 µL |
| Tampon de réaction 1X | 180µL | 80µL | 100µL | - |
| Substrat | 20µL | 20µL | 20µL | 20µL |
| Enzyme | - | 100µL | - | 100µL |

Les produits sont laissés à incuber pendant deux heures à température ambiante à l'abri de la lumière, puis la fluorescence est déterminée (Excitation 495nm; Emission: 515nm).

Chaque essai est répété trois fois. Pour chaque échantillon, on soustrait les valeurs de l'essai à blanc. Le pourcentage d'inhibition correspond au rapport Moyenne de l'activité collagénase obtenues pour chaque extrait sur Moyenne de l'activité collagénase du groupe témoin. Les résultats obtenus sont consignés dans le tableau suivant :

| Produits testés | Concentration | Fluorescence | Ecart-type | Inhibition |
|---|---|---|---|---|
| Témoin 1 | - | 101,49UA | 14,36 | - |
| 1,10-phénanthroline | 0,01% | 0,91UA | 1,07 | 99% |
| 1,10-phénanthroline | 0,001% | 24,41UA | 3,65 | 76% |
| AmB | 100 µg/ml | 49,04UA | 16,06 | 52% |
| AmB | 50µg/ml | 86,77UA | 7,98 | 15% |
| Témoin 2 | - | 110,16UA | 10,03 | - |
| 1,10-phénanthroline | 0,01% | 1,07UA | 0,94 | 99% |
| 1,10-phénanthroline | 0.001% | 37,13UA | 13,17 | 66% |
| AmE | 100µg/ml | -0,02UA | 0,06 | 100% |
| AmE | 50µml | 23,51UA | 7,52 | 79% |
| DMSO | 1% | 91,62UA | 13,07 | 17% |
| DMSO | 0,5% | 116,79 | 8,36 | -6% |

Ces résultats font apparaître que l'extrait selon l'invention (AmB) a un effet significatif à 100µg/ml sur l'inhibition de l'activité anticollagénase, quoique inférieur à celui de l'extrait brut (AmE).

### Evaluation de la phototoxicité des Extraits AmB selon l'invention et AmE selon l'état de la technique

Cette évaluation est réalisée en se fondant sur les conditions expérimentales de l'essai n°432 intitulé : Essai de phototoxicité in vitro 3T3 NRU, des lignes directrices de l'OCDE pour les essais de produits chimiques en ce qui concerne les conditions relatives à la cinétique de culture, et à la dose de limite acceptable pour le Chlopromazine (CPZ). Cette ligne directrice décrit une méthode pour évaluer le photo-cytotoxicité par la réduction relative de la viabilité des cellules exposées au produit chimique en présence ou en absence de lumière.

### Conditions expérimentales utilisées

Un pool de fibroblastes humains normaux de type Caucasien est utilisé et amplifié pendant cinq jours en flasque 75cm² dans du milieu de culture spécifique à 10% de SVF*. Les cellules sont ensuite ensemencées dans des puits de culture d'une plaque 96 puits (4 puits/condition), à 10000 cellules/puits, dans 200µL de milieu de culture spécifique à 10% de SVF (Sérum de veau foetal). 24h après l'ensemencement les cellules sont traitées avec les produits à l'essai préparés en solution EBSS (Earle's balance sait Solution) pendant une heure. A l'issue de cette incubation, les cellules sont exposées (+irr) ou non (-irr) à une dose de 1J/cm² d'UVA, en présence des produits à l'essai. A l'issue de cette exposition, les tapis cellulaires sont rincés puis remis en contact avec du milieu de culture spécifique à 2% de SVF. 24h après ce changement, la viabilité cellulaire est évaluée à l'aide de la méthode MTT (dosage colorimétrique révélé à 540nm).

Les résultats sont exprimés en pourcentage de viabilité par rapport au témoin. On en déduit les paramètres suivants:
CE₅₀: Concentration d'actif induisant 50% de toxicité (-irr) ou phototoxicité (+irr)
PIF (facteur de photo-irradiation): CE₅₀₍₋ᵢᵣᵣ₎/ CE₅₀₍₊ᵢᵣᵣ₎
PIF < 2: Aucune phototoxicité
2<PIF<5: Phototoxicité probable
PIF>5: Phototoxicité.

Les évaluations de phototoxicité pour chacun des produits testés sont consignées dans le tableau suivant:

| | CE₅₀₍₋ᵢᵣᵣ₎ | CE₅₀₍₊ᵢᵣᵣ₎ | PIF |
|---|---|---|---|
| CPZ | 22,30 | 0,25 | 89,2 |
| AmB | 261,19 | 238,95 | 1,09 |
| AmE | 47,02 | 23,04 | 2,04 |

Elles mettent en évidence que, contrairement à l'extrait brut, l'extrait AmB selon l'invention n'est pas phototoxique et montre une moindre toxicité que celle mesurée pour l'extrait brut AmE et pour la référence CPZ.

### Exemples de formulations cosmétiques contenant l'extrait AmB

### Emulsion Huile-dans-eau dermo-purifiante

| | |
|---|---|
| Eau | qsp 100% |
| Glycérine | 3% |
| Solagum^{™}AX | 0,3% |
| Montanov^{™}202 | 2% |
| Lanol^{™} 99 | 7% |
| Cétiol^{™}OE | 3% |
| Lanol^{™}P | 0,25% |
| Sepiplus^{™}400 | 0,8% |
| Euxyl^{™}PE9010 | 1% |
| Sensiva^{™}PA40 | 0,5% |
| Extrait AmB | 1% |
| Acide lactique à 20% | qs pH = 5,5 |

### Emulsion Huile-dans-eau anti-sébum

| | |
|---|---|
| Eau | qsp 100% |
| Montanov^{™}202 | 3% |
| Montanov^{™} 14 | 1,5% |
| Pelemol^{™}BB | 2% |
| Beurre de Karité | 1,5% |
| Phytosqualane | 3% |
| Huile de jojoba | 3% |
| Triglycéride C8-C10 | 3% |
| DUB ISIP | 3% |
| D,L α-tocophérol | 0,1% |
| Solagum^{™}Tara | 0,6% |
| Extrait AmB | 2% |
| Acide sorbique | 0,3% |
| Soude 48% | 0,07% |

### Sérum apaisant

| | |
|---|---|
| Sepimax^{™}Zen | 0,5% |
| Eau | qsp 100% |
| Butylène glycol | 2% |
| Aquaxyl^{™} | 2% |
| Extrait AmB | 1% |
| Montanox 20 | 1% |
| Phenoxyethanol & Ethylhexyl Glycerin | 0,80% |

SOLAGUM^{™}AX: Mélange de gomme d'acacia et de gomme de xanthane utilisé comme agent émulsionnant ;
MONTANOV^{™}202 (nom INCI : Arachidyl Alcohol & Behenyl Alcohol & Arachidyl Glucoside): Agent émulsionnant ;
LANOL^{™} 99: Isononanoate d'isononyle ;
Cétiol^{™}OE (nom INCI : Dicaprylyl ether): Phase grasse ;
LANOL^{™} P: Glycol palmitate ;
SEPIPLUS^{™}400 (nom INCI : Polyacrylate-13 & Polyisobutene & Polysorbate 20): Agent épaississant polymérique ;
EUXYL^{™}PE9010 (nom INCI: phenoxyethanol and ethylhexylglycerin): Agent conservateur ;
SENSIVA^{™}PA40 (nom INCI : Phenethyl Alcohol (and) Ethylhexylglycerin)/Agent antimicrobien ;
MONTANOV^{™}14 (nom INCI : Myristyl Alcohol & Myristyl Glucoside) : Agent émulsionnant; PELEMOL^{™}BB : Behenyl Behenate ;
SOLAGUM^{™}Tara : Gomme de Tara utilisée comme agent émulsionnant ;
SEPIMAX^{™}Zen (nom INCI : polyacrylate crosspolymer-6) : Agent épaississant, émulsionnant et stabilisant
AQUAXYL^{™} (nom INCI: Xylitylglucoside and Anhydroxylitol and Xylitol) : Composition hydratante ;
MONTANOX^{™} 20 (nom INCI : Polysorbate 20) : Agent émulsionnant de type huile-dans-eau.

## Revendications

1. Procédé pour préparer un extrait butanolique issu d'une biomasse de parties aériennes *d'Armeria maritima,* comprenant les étapes successives suivantes :
- Une étape A₀) de récolte des dites parties aériennes *d'Armeria maritima,* pour en obtenir une récolte des dites parties aériennes ;
- Une étape A₁) de séchage de ladite récolte obtenue à l'étape A₀) ;
- Une étape A₂) de broyage de ladite récolte séchée à l'étape A₁), pour obtenir un broyat de ladite récolte séchée ;
- si nécessaire ou si désiré, une étape A₃) de pulvérisation du broyat obtenu à l'étape A₂), pour obtenir une poudre de ladite récolte séchée ;
- Au moins une étape A) de macération dans une solution hydro-éthanolique de ratio volumique éthanol/eau égal à 80/20, de ladite poudre obtenue à l'étape A₃, pour en obtenir une suspension hydro-éthanolique ;
- Au moins une étape B) de filtration de ladite suspension hydro-éthanolique obtenue à l'étape A), pour en séparer un extrait hydro-éthanolique ;
- Une étape C) de séchage dudit extrait hydro-éthanolique obtenu à l'étape B), pour en obtenir un résidu sec ;
- Une étape D) de solubilisation dans l'eau dudit résidu sec obtenu à l'étape C), pour obtenir une phase aqueuse φ₀ ;
- Au moins une étape E₁) d'extraction liquide-liquide par mise en contact de ladite phase aqueuse φ₀ obtenue à l'étape D), avec du cyclohexane en un ratio volumique cyclohexane/phase aqueuse φ₀ égal à 2/3, pour obtenir après séparation des phases non miscibles, une nouvelle phase aqueuse φ₁ ;
- Au moins une étape E₂ₐ) d'extraction liquide-liquide par mise en contact de ladite phase aqueuse φ₁ obtenue à l'étape E₁), avec du dichlorométhane en un ratio volumique dichlorométhane/phase aqueuse φ₁ égal à 2/3, pour obtenir après séparation des phases non miscibles, une nouvelle phase aqueuse φ₂ₐ ;
- Au moins une étape E_{2b}) d'extraction liquide-liquide par mise en contact de ladite phase aqueuse φ₂ₐ obtenue à l'étape E₂ₐ), avec de l'acétate d'éthyle en un ratio volumique acétate d'éthyle/phase aqueuse φ₂ₐ égal à 2/3, pour obtenir après séparation des phases non miscibles, une nouvelle phase aqueuse φ_{2b} ;
- Au moins une étape E₃) d'extraction liquide-liquide par mise en contact de ladite phase aqueuse φ_{2b} obtenue à l'étape E_{2b}), avec du 1-butanol saturé en eau en un ratio volumique 1-butanol/phase aqueuse φ_{2b}, égal à 2/3, pour obtenir après séparation des phases non miscibles, ledit extrait butanolique attendu et une nouvelle phase aqueuse φ₃.

2. Extrait butanolique d'une biomasse d'*Armeria maritima*, obtenu par le procédé comprenant les étapes successives suivantes :
- Une étape A₀) de récolte des dites parties aériennes d'*Armeria maritima*, pour en obtenir une récolte des dites parties aériennes ;
- Une étape A₁) de séchage de ladite récolte obtenue à l'étape A₀) ;
- Une étape A₂) de broyage de ladite récolte séchée à l'étape A₁), pour obtenir un broyat de ladite récolte séchée ;
- Si nécessaire ou si désiré, une étape A₃) de pulvérisation du broyat obtenu à l'étape A₂), pour obtenir une poudre de ladite récolte séchée ;
- Au moins une étape A) de macération dans une solution hydro-éthanolique de ratio volumique éthanol/eau égal à 80/20, de ladite poudre obtenue à l'étape A₃, pour en obtenir une suspension hydro-éthanolique ;
- Au moins une étape B) de filtration de ladite suspension hydro-éthanolique obtenue à l'étape A), pour en séparer un extrait hydro-éthanolique ;
- Une étape C) de séchage dudit extrait hydro-éthanolique obtenu à l'étape B), pour en obtenir un résidu sec ;
- Une étape D) de solubilisation dans l'eau dudit résidu sec obtenu à l'étape C), pour obtenir une phase aqueuse φ₀ ;
- Au moins une étape E₁) d'extraction liquide-liquide par mise en contact de ladite phase aqueuse φ₀ obtenue à l'étape D), avec du cyclohexane en un ratio volumique cyclohexane/phase aqueuse φ₀ égal à 2/3, pour obtenir après séparation des phases non miscibles, une nouvelle phase aqueuse φ₁ ;
- Au moins une étape E₂ₐ) d'extraction liquide-liquide par mise en contact de ladite phase aqueuse φ₁ obtenue à l'étape E₁), avec du dichlorométhane en un ratio volumique dichlorométhane/phase aqueuse φ₁ égal à 2/3, pour obtenir après séparation des phases non miscibles, une nouvelle phase aqueuse φ₂ₐ ;
- Au moins une étape E_{2b}) d'extraction liquide-liquide par mise en contact de ladite phase aqueuse φ₂ₐ obtenue à l'étape E₂ₐ), avec de l'acétate d'éthyle en un ratio volumique acétate d'éthyle/phase aqueuse φ₂ₐ égal à 2/3, pour obtenir après séparation des phases non miscibles, une nouvelle phase aqueuse φ_{2b} ;
- Au moins une étape E₃) d'extraction liquide-liquide par mise en contact de ladite phase aqueuse φ_{2b} obtenue à l'étape E_{2b}), avec du 1-butanol saturé en eau en un ratio volumique 1-butanol/phase aqueuse φ_{2b}, égal à 2/3, pour obtenir après séparation des phases non miscibles, ledit extrait butanolique attendu et une nouvelle phase aqueuse φ₃.

3. Utilisation de l'extrait butanolique tel que défini à la revendication 2, comme agent actif cosmétique anti-âge.

4. Composition cosmétique (C₁), à usage topique comprenant au moins un excipient cosmétiquement acceptable et une quantité efficace de l'extrait butanolique tel que défini à la revendication 2.

## Patentansprüche

1. Verfahren zur Herstellung eines butanolischen Extrakts aus einer Biomasse von oberirdischen Teilen von *Armeria maritima,* umfassend die folgenden aufeinanderfolgenden Schritte:
- einen Schritt A₀) der Ernte der oberirdischen Teile von *Armeria maritima,* um daraus eine Ernte der oberirdischen Teile zu gewinnen;
- einen Schritt A₁) des Trocknens der im Schritt A₀) erhaltenen Ernte;
- einen Schritt A₂) des Zerkleinerns der im Schritt A₁) getrockneten Ernte, um ein Mahlgut der getrockneten Ernte zu erhalten;
- falls notwendig oder gewünscht, einen Schritt A₃) der Pulverisierung des im Schritt A₂) erhaltenen Mahlguts, um ein Pulver der getrockneten Ernte zu erhalten;
- mindestens einen Schritt A) der Mazeration in einer wässrig-ethanolischen Lösung mit einem Volumenverhältnis Ethanol/Wasser gleich 80/20 des im Schritt A₃ erhaltenen Pulvers, um daraus eine wässrigethanolische Suspension zu erhalten;
- mindestens einen Schritt B) der Filtration der im Schritt A) erhaltenen Suspension, um daraus einen wässrig-ethanolischen Extrakt abzutrennen;
- einen Schritt C) des Trocknens des im Schritt B) erhaltenen wässrig-ethanolischen Extrakts, um daraus einen trockenen Rückstand zu erhalten;
- einen Schritt D) des Solubilisierens des im Schritt C) erhaltenen trockenen Rückstands in Wasser, um eine wässrige Phase φ₀ zu erhalten;
- mindestens einen Schritt E₁) der Flüssig-Flüssig-Extraktion durch Inkontaktbringen der im Schritt D) erhaltenen wässrigen Phase φ₀ mit Cyclohexan in einem Volumenverhältnis Cyclohexan/wässrige Phase φ₀ gleich 2/3, um nach Trennung der nicht mischbaren Phasen eine neue wässrige Phase φ₁ zu erhalten;
- mindestens einen Schritt E₂ₐ) der Flüssig-Flüssig-Extraktion durch Inkontaktbringen der im Schritt E₁) erhaltenen wässrigen Phase φ₁ mit Dichlormethan in einem Volumenverhältnis Dichlormethan/wässrige Phase φ₁ gleich 2/3, um nach Trennung der nicht mischbaren Phasen eine neue wässrige Phase φ₂ₐ zu erhalten;
- mindestens einen Schritt E_{2b}) der Flüssig-Flüssig-Extraktion durch Inkontaktbringen der im Schritt E₂ₐ) erhaltenen wässrigen Phase φ₂ₐ mit Ethylacetat in einem Volumenverhältnis Ethylacetat/wässrige Phase φ₂ₐ gleich 2/3, um nach Trennung der nicht mischbaren Phasen eine neue wässrige Phase φ_{2b} zu erhalten;
- mindestens einen Schritt E₃) der Flüssig-Flüssig-Extraktion durch Inkontaktbringen der im Schritt E_{2b}) erhaltenen wässrigen Phase φ_{2b} mit wassergesättigtem 1-Butanol in einem Volumenverhältnis 1-Butanol/wässrige Phase φ_{2b} gleich 2/3, um nach Trennung der nicht mischbaren Phasen den erwarteten butanolischen Extrakt und eine neue wässrige Phase φ₃ zu erhalten.

2. Butanolischer Extrakt einer Biomasse von *Armeria maritima,* der durch das Verfahren erhalten wird, das die folgenden aufeinanderfolgenden Schritte umfasst:
- einen Schritt A₀) der Ernte der oberirdischen Teile von *Armeria maritima*, um daraus eine Ernte der oberirdischen Teile zu gewinnen;
- einen Schritt A₁) des Trocknens der im Schritt A₀) erhaltenen Ernte;
- einen Schritt A₂) des Zerkleinerns der im Schritt A₁) getrockneten Ernte, um ein Mahlgut der getrockneten Ernte zu erhalten;
- falls notwendig oder gewünscht, einen Schritt A₃) der Pulverisierung des im Schritt A₂) erhaltenen Mahlguts, um ein Pulver der getrockneten Ernte zu erhalten;
- mindestens einen Schritt A) der Mazeration in einer wässrig-ethanolischen Lösung mit einem Volumenverhältnis Ethanol/Wasser gleich 80/20 des im Schritt A₃ erhaltenen Pulvers, um daraus eine wässrigethanolische Suspension zu erhalten;
- mindestens einen Schritt B) der Filtration der im Schritt A) erhaltenen Suspension, um daraus einen wässrig-ethanolischen Extrakt abzutrennen;
- einen Schritt C) des Trocknens des im Schritt B) erhaltenen wässrig-ethanolischen Extrakts, um daraus einen trockenen Rückstand zu erhalten;
- einen Schritt D) des Solubilisierens des im Schritt C) erhaltenen trockenen Rückstands in Wasser, um eine wässrige Phase φ₀ zu erhalten;
- mindestens einen Schritt E₁) der Flüssig-Flüssig-Extraktion durch Inkontaktbringen der im Schritt D) erhaltenen wässrigen Phase φ₀ mit Cyclohexan in einem Volumenverhältnis Cyclohexan/wässrige Phase φ₀ gleich 2/3, um nach Trennung der nicht mischbaren Phasen eine neue wässrige Phase φ₁ zu erhalten;
- mindestens einen Schritt E₂ₐ) der Flüssig-Flüssig-Extraktion durch Inkontaktbringen der im Schritt E₁) erhaltenen wässrigen Phase φ₁ mit Dichlormethan in einem Volumenverhältnis Dichlormethan/wässrige Phase φ₁ gleich 2/3, um nach Trennung der nicht mischbaren Phasen eine neue wässrige Phase φ₂ₐ zu erhalten;
- mindestens einen Schritt E_{2b}) der Flüssig-Flüssig-Extraktion durch Inkontaktbringen der im Schritt E₂ₐ) erhaltenen wässrigen Phase φ₂ₐ mit Ethylacetat in einem Volumenverhältnis Ethylacetat/wässrige Phase φ₂ₐ gleich 2/3, um nach Trennung der nicht mischbaren Phasen eine neue wässrige Phase φ_{2b} zu erhalten;
- mindestens einen Schritt E₃) der Flüssig-Flüssig-Extraktion durch Inkontaktbringen der im Schritt E_{2b}) erhaltenen wässrigen Phase φ_{2b} mit wassergesättigtem 1-Butanol in einem Volumenverhältnis 1-Butanol/wässrige Phase φ_{2b} gleich 2/3, um nach Trennung der nicht mischbaren Phasen den erwarteten butanolischen Extrakt und eine neue wässrige Phase φ₃ zu erhalten.

3. Verwendung des butanolischen Extrakts nach Anspruch 2 als kosmetischen Anti-Aging-Wirkstoff.

4. Kosmetische Zusammensetzung (C₁) zur topischen Verwendung, umfassend mindestens einen kosmetisch akzeptablen Exzipienten und eine wirksame Menge des butanolischen Extrakts nach Anspruch 2.

## Claims

1. Process for preparing a butanolic extract originating from a biomass of aerial parts of *Armeria maritima,* comprising the following successive steps:
- a step A₀) of harvesting said aerial parts of *Armeria maritima,* in order to obtain therefrom a harvest of said aerial parts;
- a step A₁) of drying said harvest obtained in step A₀) ;
- a step A₂) of milling said harvest dried in step A₁), in order to obtain a milled material of said dried harvest;
- if necessary or if desired, a step A₃) of spraying the milled material obtained in step A₂), in order to obtain a powder of said dried harvest;
- at least one step A) of macerating, in an aqueous-ethanolic solution with an ethanol/water volume ratio equal to 80/20, said powder obtained in step A₃, in order to obtain an aqueous-ethanolic suspension therefrom;
- at least one step B) of filtering said aqueous-ethanolic suspension obtained in step A), in order to separate an aqueous-ethanolic extract therefrom;
- a step C) of drying said aqueous-ethanolic extract obtained in step B), in order to obtain a dry residue therefrom;
- a step D) of dissolving in water said dry residue obtained in step C), in order to obtain an aqueous phase φ₀;
- at least one step E₁) of liquid-liquid extraction by bringing said aqueous phase φ₀ obtained in step D) into contact with cyclohexane in a cyclohexane/aqueous phase φ₀ volume ratio equal to 2/3, in order to obtain, after separation of the immiscible phases, a new aqueous phase φ₁;
- at least one step E₂ₐ) of liquid-liquid extraction by bringing said aqueous phase φ₁ obtained in step E₁) into contact with dichloromethane in a dichloromethane/aqueous phase φ₁ volume ratio equal to 2/3, in order to obtain, after separation of the immiscible phases, a new aqueous phase φ₂ₐ;
- at least one step E_{2b}) of liquid-liquid extraction by bringing said aqueous phase φ₂ₐ obtained in step E₂ₐ) into contact with ethyl acetate in an ethyl acetate/aqueous phase φ₂ₐ volume ratio equal to 2/3, in order to obtain, after separation of the immiscible phases, a new aqueous phase φ_{2b};
- at least one step E₃) of liquid-liquid extraction by bringing said aqueous phase φ_{2b} obtained in step E_{2b}) into contact with water-saturated 1-butanol in a 1-butanol/aqueous phase φ_{2b} volume ratio equal to 2/3, in order to obtain, after separation of the immiscible phases, said expected butanolic extract and a new aqueous phase φ₃.

2. Butanolic extract of a biomass of *Armeria maritima,* obtained by the process comprising the following successive steps:
- a step A₀) of harvesting said aerial parts of *Armeria maritima,* in order to obtain therefrom a harvest of said aerial parts;
- a step A₁) of drying said harvest obtained in step A₀) ;
- a step A₂) of milling said harvest dried in step A₁), in order to obtain a milled material of said dried harvest;
- if necessary or if desired, a step A₃) of spraying the milled material obtained in step A₂), in order to obtain a powder of said dried harvest;
- at least one step A) of macerating, in an aqueous-ethanolic solution with an ethanol/water volume ratio equal to 80/20, said powder obtained in step A₃, in order to obtain an aqueous-ethanolic suspension therefrom;
- at least one step B) of filtering said aqueous-ethanolic suspension obtained in step A), in order to separate an aqueous-ethanolic extract therefrom;
- a step C) of drying said aqueous-ethanolic extract obtained in step B), in order to obtain a dry residue therefrom;
- a step D) of dissolving in water said dry residue obtained in step C), in order to obtain an aqueous phase φ₀;
- at least one step E₁) of liquid-liquid extraction by bringing said aqueous phase φ₀ obtained in step D) into contact with cyclohexane in a cyclohexane/aqueous phase φ₀ volume ratio equal to 2/3, in order to obtain, after separation of the immiscible phases, a new aqueous phase φ₁;
- at least one step E₂ₐ) of liquid-liquid extraction by bringing said aqueous phase φ₁ obtained in step E₁) into contact with dichloromethane in a dichloromethane/aqueous phase φ₁ volume ratio equal to 2/3, in order to obtain, after separation of the immiscible phases, a new aqueous phase φ₂ₐ;
- at least one step E_{2b}) of liquid-liquid extraction by bringing said aqueous phase φ₂ₐ obtained in step E₂ₐ) into contact with ethyl acetate in an ethyl acetate/aqueous phase φ₂ₐ volume ratio equal to 2/3, in order to obtain, after separation of the immiscible phases, a new aqueous phase φ_{2b};
- at least one step E₃) of liquid-liquid extraction by bringing said aqueous phase φ_{2b} obtained in step E_{2b}) into contact with water-saturated 1-butanol in a 1-butanol/aqueous phase φ_{2b} volume ratio equal to 2/3, in order to obtain, after separation of the immiscible phases, said expected butanolic extract and a new aqueous phase φ₃.

3. Use of the butanolic extract as defined in Claim 2, as cosmetic anti-ageing active agent.

4. Cosmetic composition (C₁) for topical use, comprising at least one cosmetically acceptable excipient and an effective amount of the butanolic extract as defined in Claim 2.
